(19) Europäisches Patentamt
European Patent Office
Office européen des brevets

(11) Publication number: **0 027 877**
**B1**

(12) **EUROPEAN PATENT SPECIFICATION**

(45) Date of publication of patent specification: **13.07.83**

(21) Application number: **80105206.9**

(22) Date of filing: **02.09.80**

(51) Int. Cl.³: **C 07 C 133/10,**
C 07 D 233/52,
C 07 D 243/04,
A 61 K 31/175,
A 61 K 31/395

(54) **5,8-dihydroxy-1,4-bis(guanidinyl-amino)anthraquinones.**

(30) Priority: **24.10.79 US 87908**

(43) Date of publication of application:
**06.05.81 Bulletin 81/18**

(45) Publication of the grant of the patent:
**13.07.83 Bulletin 83/28**

(84) Designated Contracting States:
**BE CH DE FR GB IT LI NL SE**

(56) References cited:
**GB - A - 2 004 293**
**GB - A - 2 009 171**

(73) Proprietor: **AMERICAN CYANAMID COMPANY**
**1937 West Main Street P.O. Box 60**
**Stamford Connecticut 06904 (US)**

(72) Inventor: **Murdock, Keith Chadwick**
**15 Birch Street**
**Pearl River, New York 10965 (US)**

(74) Representative: **Diehl, Hermann O., Dr. et al,**
**Diehl & Kressin Flüggenstrasse 17**
**D-8000 München 19 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 027 877 B1

5,8-Dihydroxy-1,4-bis(guanidinyl-amino)anthraquinones

This invention relates to new organic compounds and, more particularly, is concerned with novel 1,4-bis(guanidinylamino)-5,8-dihydroxyanthraquinones which may be represented by the following general formula:

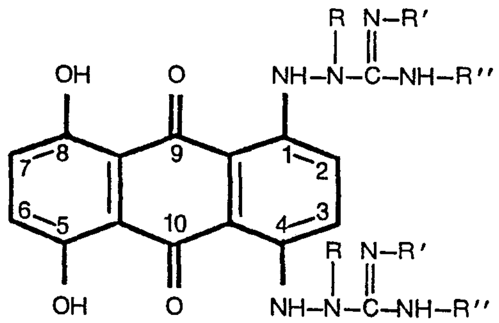

(I)

wherein R, R′ and R″ are selected from the group consisting of hydrogen and straight or branched chain lower alkyl ($C_1$—$C_4$) groups and R′ and R″ taken together is alkylene $-(CH_2)_n-$, where n is an integer from 2 to 4, inclusive.

Also included within the purview of the present invention are the leuco bases and tautomers thereof which may be represented by the following general formula:

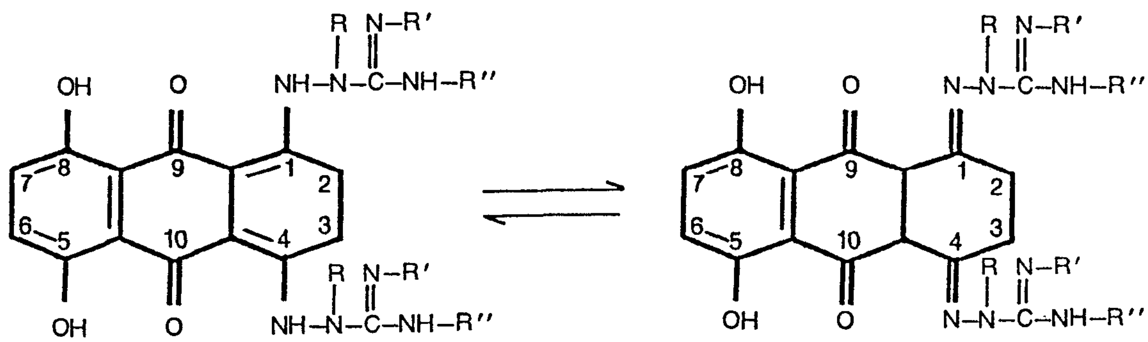

(II, Leuco Bases) (III, Tautomeric Form)

where R, R′, R″ and n are as previously defined, and pharmaceutically acceptable acid-addition salts thereof.

Similar anthraquinone derivatives are known as anti-tumor agents from GB 2004293 A. These compounds are not as active as the compounds of the present invention.

The novel compounds of the present invention are obtainable as reddish brown to blue crystalline materials having characteristic melting points and absorption spectra and which may be purified by leaching with lower alkanols since many of the free bases are insoluble in water and some of them are insoluble in most organic solvents. The organic bases of this invention (I, II and III) form acid-addition salts with a variety of pharmacologically acceptable organic and inorganic salt-forming reagents. Thus, acid-addition salts, formed by admixture of the organic free base with an acid, suitably in a neutral solvent, are formed with such acids as sulfuric, phosphoric, hydrochloric, hydrobromic, sulfamic, citric, lactic, malic, succinic, tartaric, acetic, benzoic, gluconic, ascorbic, and the like. For purposes of this invention, the free bases are equivalent to their acid-addition salts. The acid-addition salts of the organic bases of the present invention are, in general, crystalline solids, relatively soluble in water, methanol and ethanol but relatively insoluble in non-polar organic solvents such as diethyl ether, benzene, toluene, and the like.

The novel compounds of the present invention may be readily prepared in accordance with the following reaction scheme:

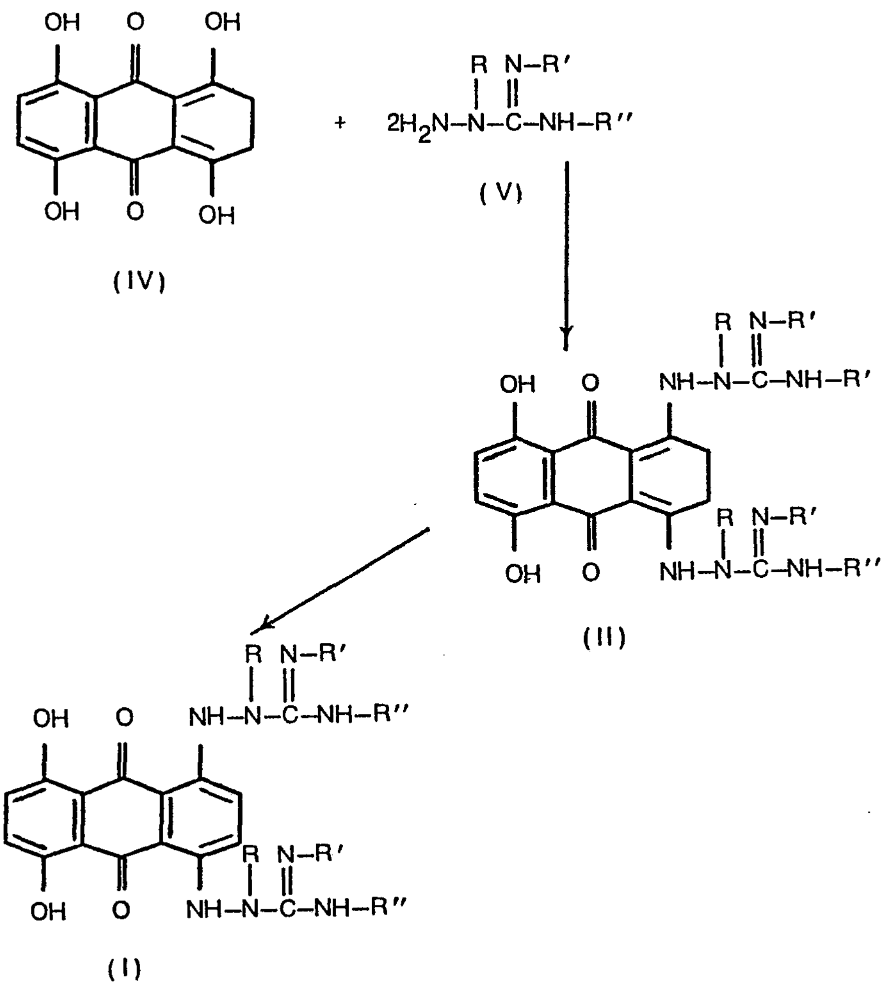

(IV) (V) (II) (I)

wherein R, R', R" and n are as hereinabove defined. In accordance with this reaction scheme, leuco 1,4,5,8-tetrahydroxyanthraquinone (IV) is condensed with an appropriate aminoguanidine (V) in a solvent such as N,N,N'N'-tetramethylethylenediamine, 2-methoxyethanol, methanol, ethanol, water, dimethylformamide, or mixtures thereof at from about 40°C. to about 60°C. under an atmosphere of nitrogen for one or more hours to produce the corresponding leuco bases (II). The leuco bases (II) may be readily oxidized to the fully aromatic derivatives (I) by a variety of methods such as air oxidation or treatment with chloranil, hydrogen peroxide or sodium perborate.

The novel compounds described herein are useful as chelating, complexing or sequestering agents. The complexes formed with polyvalent metal ions are particularly stable and usually soluble in various organic solvents. These properties, of course, render them useful for a variety of purposes wherein metal ion contamination presents a problem; e.g., as stabilizers in various organic systems such as saturated and unsaturated lubricating oils and hydrocarbons, fatty acids and waxes, wherein transition metal ion contamination accelerates oxidative deterioration and color formation. They are further useful in analyses of polyvalent metal ions which may be complexed or extracted by these materials and as metal carriers. Other uses common to sequestering agents are also apparent for these compounds. In addition, the leuco bases (II) are useful as intermediates in the preparation of the fully aromatic derivatives (I).

The novel compounds of the present invention also possess the property of inhibiting the growth of transplanted mouse tumors as established by the following tests.

Lymphocytic leukemia P388 test

The animals used are DBA/2 mice all of one sex, weighing a minimum of 17 g. and all within a 3 g. weight range. There are 5 or 6 animals per test group. The tumor transplant is by intraperitoneal injection of 0.1 ml. of dilute ascitic fluid containing $10^6$ cells of lymphocytic leukemia P388. The test compounds are administered intraperitoneally on days one, 5 and 9 (relative to tumor inoculation) at various doses. The animals are weighed and survivors are recorded on a regular basis for 30 days. The median survival time and the ratio of survival time for treated (T)/control (C) animals are calculated. The positive control compound is 5-fluorouracil given as a 60 mg./kg. injection. The results of this test with

representative compounds of the present invention appear in Table I. The criterion for efficacy is T/C x 100 ≥ 125%.

TABLE I

Lymphocytic Leukemia P388 Test

| Compound | Dose (mg. /kg.) | Median Survival Time (Days) | T/C × 100 (Percent) |
|---|---|---|---|
| 1,1'-(5,8-Dihydroxy-1,4-anthraquinonylene-diimino)diguanidine dihydrochloride | 200 | 18.0 | 164 |
| | 100 | 16.0 | 145 |
| | 50 | 16.0 | 145 |
| | 25 | 15.0 | 136 |
| | 12 | 15.0 | 136 |
| Control | | 11.0 | |
| 5-Fluorouracil | 60 | 21.5 | 195 |
| Leuco-5,8-dihydroxy-1,4-bis[2-(2-imidazolin-2-yl)hydrazino]-anthraquinone | 200 | 23.0 | 209 |
| | 100 | 19.0 | 173 |
| | 50 | 18.0 | 164 |
| | 25 | 17.0 | 155 |
| | 12 | 17.0 | 155 |
| Control | | 11.0 | |
| 5-Fluorouracil | 60 | 20.5 | 186 |

Melanotic melanoma B16

The animals used are C57BC/6 mice, all of the same sex, weighing a minimum of 17 g. and all within a 3 g. weight range. There are normally 10 animals per test group. A one-gram portion of melanotic melanoma B16 tumor is homogenized in 10 ml. of cold balanced salt solution and a 0.5 ml. aliquot of the homogenate is implanted intraperitoneally into each of the test mice. The test compounds are administered intraperitoneally on days one through 9 (relative to tumor inoculation) at various doses. The animals are weighed and survivors are recorded on a regular basis for 60 days. The median survival time and the ratio of survival time for treated (T)/control (C) animals are calculated. The positive control compound is 5-fluorouracil given as a 20 mg./kg. injection. The results of this test with representative compounds of the present invention appear in Table II. The criterion for efficacy is T/C x 100 ≥ 125%.

TABLE II

Melanotic Melanoma B16 Test

| Compound | Dose (Mg. /kg.) | Median Survival Time (Days) | T/C × 100 (Percent) |
|---|---|---|---|
| 1,1'-(5,8-Dihydroxy-1,4-anthraquinonylene-diimino)diguanidine dihydrochloride | 100 | 33.0 | 220 |
| | 50 | 32.0 | 213 |
| | 25 | 29.0 | 193 |
| | 12 | 23.5 | 158 |
| | 6 | 22.0 | 147 |
| Control | | 15.0 | |
| 5-Fluorouracil | 20 | 27.5 | 183 |
| Leuco-5,8-dihydroxy-1,4-bis[2-(2-imidazolin-2-yl)hydrazino]-anthraquinone | 50 | 32.5 | 197 |
| | 25 | 28.0 | 170 |
| | 12 | 27.0 | 164 |
| | 6 | 26.5 | 161 |
| Control | | 16.5 | |
| 5-Fluorouracil | 20 | 16.5 | 100 |

Lymphocytic leukemia L1210 test

The procedure is the same as for the Lymphocytic leukemia P388 test except that the tumor transplant consists of lymphocytic leukemia L1210 inoculated at a concentration of $10^5$ cells/mouse with a mean survival time being calculated, and the test compound is administered only on day one. The results of this test with a representative compound of this invention appear in Table III below. The criterion for efficacy is T/C × 100 ≥ 125%.

TABLE III

Lymphocytic Leukemia L1210 Test

| Compound | Dose (mg. /kg.) | Median Survival Time (Days) | T/C × 100 (Percent) |
|---|---|---|---|
| 1,1'-(5,8-Dihydroxy-1,4-anthraquinonylene-diimino)diguanidine dihydrochloride | 200 | 12.8 | 165 |
| | 100 | 10.8 | 139 |
| | 50 | 10.8 | 139 |
| | 25 | 10.3 | 132 |
| | 12.5 | 10.0 | 128 |
| Control | | 7.8 | |
| 5-Fluorouracil | 60 | 20.8 | 267 |

The novel compounds of the present invention can be used as active ingredients of therapeutic compositions of matter useful for ameliorating cancer diseases in mammals and containing certain 1,4-bis(guanidinylamino)-5,8-dihydroxyanthraquinones (or the leuco bases and non-toxic acid-addition salts thereof) which may be represented by the following structural formula:

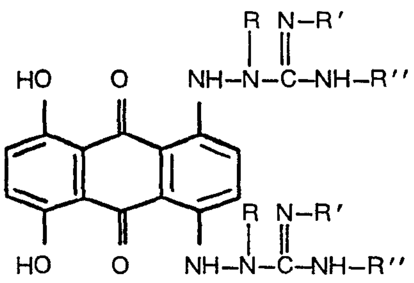

(I)

wherein R, R′ and R″ may be selected from the group comprising hydrogen and straight or branched chain lower alkyl ($C_1$—$C_4$), R′ and R″ taken together may also be alkylene, $-(CH_2)_n-$, where n is an integer from 2 to 4, inclusive.

The active ingredients of the therapeutic compositions and the novel compounds of the present invention inhibit transplanted mouse tumor growth and induce regression and/or palliation of leukemia and related cancers in mammals when administered in amounts ranging from about 5 mg. to about 1.0 g. per kilogram of body weight per day. A preferred dosage regimen for optimum results would be from about 5 mg. to about 100 mg. per kilogram of body weight per day, and such dosage units are employed that a total of from about 350 mg. to about 7.0 g. of the active compound for a subject of about 70 kg. of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. A decided practical advantage is that the active compound may be administered in any convenient manner such as by the intravenous, intramuscular, or subcutaneous routes.

The active compounds may be administered parenterally or intraperitoneally. Solutions of the active compound as a free base or pharmacologically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art

of compounding such an active material for the treatment of disease in living subjects having a diseased condition in which bodily health is impaired as herein disclosed in detail.

The principal active ingredient is compound for convenient and effective administration in effective amounts with a suitable pharmaceutically-acceptable carrier in dosage unit form as hereinbefore disclosed. A unit dosage form can, for example, contain the principal active compound in amounts ranging from about 0.1 to about 500 mg., with from about one to about 500 mg. being preferred. Expressed in proportions, the active compound is generally present in from about one to about 500 mg./ml. of carrier. In the case of compositions containing supplementary active ingredients, the dosages are determined by reference to the usual dose and manner of administration of the said ingredients.

Regression and palliation of cancers are attained, for example, using intraperitoneal administration. A single intravenous dosage or repeated daily dosages can be administered. Daily dosages up to about 5 or 10 days are often sufficient. It is also possible to dispense one daily dosage or one dose on alternate or less frequent days. As can be seen from the dosage regimens, the amount of principal active ingredient administered is a sufficient amount to aid regression and palliation of the leukemia or the like, in the absence of excessive deleterious side effects of a cytotoxic nature to the hosts harboring the cancer. As used herein, cancer disease means blood malignancies such as leukemia, as well as other solid and non-solid malignancies such as the melanocarcinomas, lung carcinomas, and mammary tumors. By regression and palliation is meant arresting or retarding the growth of the tumor or other manifestation of the disease compared to the course of the disease in the absence of treatment.

This invention will be described in greater detail in conjunction with the following specific Examples.

### Example 1

1,1′-(5,8-Dihydroxy-1,4-anthraquinonylenediimino)diquanidine dihydrochloride

A mixture of 19.70 g. of aminoguanidine sulfate and 16.0 ml. of 10*N* aqueous sodium hydroxide is stirred and triturated until all the lumps are gone. The mixture is diluted with 75 ml. of ethanol, chilled in an ice bath and dried for one hour with 90.0 g. of 3A molecular sieves. The solids are removed by filtration and are washed sparingly with two 35 ml. portions of ethanol. The filtrate and washings are combined and de-aerated by bubbling nitrogen through for 15 minutes, then 10.97 g. of leuco-1,4,5,8-tetrahydroxyanthraquinone is added under nitrogen. The mixture is stirred and heated under nitrogen in an oil bath at 50—51°C. for one hour, then is allowed to cool for 16 hours. The mixture is chilled and the solid is collected by filtration and washed with cold ethanol to give 13.87 g. of a black solid which is the 2,3-dihydro derivative of the title product.

A stirred suspension of 11.60 g. of the 2,3-dihydro compound is oxidized by adding it to 7.50 g. of chloranil in 200 ml. of 2-methoxyethanol, then chilling with an ice bath during the dropwise addition of 15.0 ml. of 8*N* hydrochloric acid in ethanol. The mixture is stirred for 16 hours at room temperature, then is thinned with diethyl ether. The solid is collected and washed with tetrahydrofuran to give 12.53 g. of a dark purple solid. A 10.0 g. portion of the purple solid is pulverized, stirred in 300 ml. of water at 23°C. for one hour, then centrifuged. The supernatant solution is decanted and filtered. The filtrate is freeze dried to yield 7.2 g. of the product of the Example as a red-brown solid.

### Example 2

Leuco-5,8-dihydroxy-1,4-bis[2-(2-imidazolin-2-yl)hydrazino]anthraquinone

A solution of 3.94 g. of 2-hydrazino-2-imidazoline hydrochloride (U.S. Patent No. 3,931,152) in 50 ml. of methanol is converted to the free base by passing it through a column containing 15 g. of a polystyrene quaternary ammonium hydroxide resin pre-washed with methanol. The sample is washed through the column with additional methanol. Evaporation of the eluate and washes gives 3.3 g. of a tan oil. A filtered solution of this oil in 40 ml. of N,N,N′-N′-tetramethylethylenediamine:ethanol (1:1) is de-aerated by bubbling nitrogen through it for 15 minutes, then 2.02 g. of leuco-1,4,5,8-tetrahydroxyanthraquinone is added under nitrogen. The resulting mixture is stirred under nitrogen and heated in an oil bath at 50—52°C. for one hour. The mixture is stored under nitrogen for 48 hours, the resulting solid is collected by filtration, washed once with N,N,N′-N′-tetramethylethylenediamine and 3 times with ethanol to give 3.03 g. of the product of the Example as a red-brown solid.

### Example 3

Leuco-5,8-dihydroxy-1,4-bis[2-(2-imidazolin-2-yl)-2-methylhydrazino]anthraquinone

A solution of 48.8 g of 2-methylthio-2-imidazoline hydroiodide and 10.0 g. of methylhydrazine in 200 ml. of ethanol is heated at reflux for several hours, clarified and then cooled at −10°C. The precipitate is collected, washed with diethyl ether and dried, giving 2-(1-methylhydrazine)imidazoline hydroiodide.

This hydroiodide is converted to the free base and then allowed to react with one half of that molar amount of leuco-1,4,5,8-tetrahydroxyanthraquinone as in Example 2 to give the title compound as a dark solid.

Example 4

Leuco-5,8-dihydroxy-1,4-bis[2-(4,5,6,7-tetrahydro-1H-1,3-diazepin-2-yl)hydrazine]anthraquinone

A 6.41 g. amount of 2-hydrazine-4,5,6,7-tetrahydro-1H-1,3-diazepine hydroiodide (U.S. Patent No. 3,931,152) is converted to the free base and allowed to react with 2.02 g. of leuco-1,4,5,8-tetrahydroxyanthraquinone as in Example 2, to give the title compound as a dark solid.

Example 5

1,1'-(5,8-Dihydroxy-1,4-anthraquinonylenediimino)-3,3'-dimethyldiguanidine dihydrochloride

Processing of 1-amino-3-methylguanidine hydroiodide as in Example 1 gives the title compound.

Example 6

1,1'-(5,8-Dihydroxy-1,4-anthraquinonylenediimino)-3,3'-di(1-butyl)diguanidine dihydrochloride

The title compound is obtained from 1-amino-3-(1-butyl)guanidine hydroiodide [Short, *et al.,* J. Med. Chem., *6,* 275 (1963)] by the procedure of Example 1.

**Claims**

1. A compound selected from the group consisting of those of the formula:

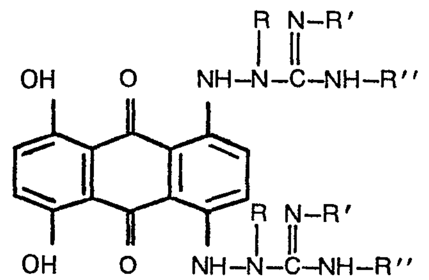

(I)

wherein R, R' and R" are selected from the group consisting of hydrogen and straight or branched chain lower alkyl ($C_1$—$C_4$) groups and R' and R" taken together is alkylene $-(CH_2)_n-$, where n is an integer from 2 to 4, inclusive, and pharmaceutically acceptable acid-addition salts thereof.

2. A compound selected from the group consisting of those of the formula:

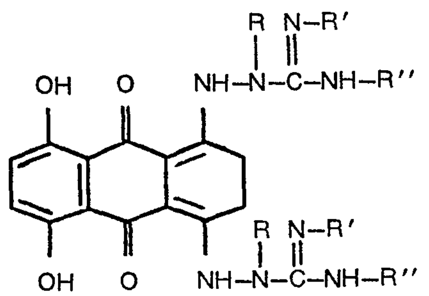

(II)

wherein R, R' and R" are selected from the group consisting of hydrogen and straight or branched chain lower alkyl ($C_1$—$C_4$) groups and R' and R" taken together is alkylene $-(CH_2)_n-$, where n is an integer from 2 to 4, inclusive, and pharmaceutically acceptable acid-addition salts thereof.

3. The compound according to Claim 1, 1,1'-(5,8-dihydroxy-1,4-anthraquinonylenediimino)diguanidine dihydrochloride.

4. The compound according to claim 1, 1,1'-(5,8-dihydroxy-1,4-anthraquinonylenediimino)-3,3'-dimethyldiguanidine dihydrochloride.

5. The compound according to Claim 1, 1,1'-(5,8-dihydroxy-1,4-anthraquinonylenediimino)-3,3'-di(1-butyl)-diguanidine dihydrochloride.

6. The compound according to Claim 2, leuco-5,8-dihydroxy-1,4-bis[2-(2-imidazolin-2-yl)hydrazino]anthraquinone.

7. The compound according to Claim 2, leuco-5,8-dihydroxy-1,4-bis[2-(2-imidazolin-2-yl)-2-methylhydrazino]anthraquinone.

8. The compound according to Claim 2, leuco-5,8-dihydroxy-1,4-bis[2-(4,5,6,7-tetrahydro-1H-1,3-diazepin-2-yl)hydrazino]anthraquinone.

**Patentansprüche**

1. Verbindung der allgemeinen Formel I

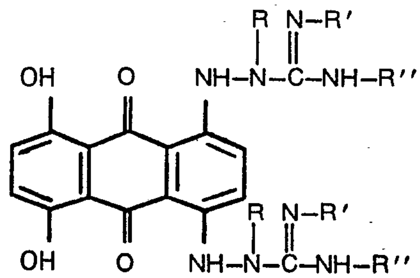

(I)

worin R, R' und R'' ausgewählt sind aus der Gruppe bestehend aus einem Wasserstoffatom und geradkettigen oder verzweigtkettigen $C_1$—$C_2$-Alkylgruppen und R' und R'' zusammen für eine Alkylengruppe $-(CH_2)_n$, worin n eine ganze Zahl von 2 bis 4 bedeutet, stehen, und die pharmazeutisch verträglichen Säureadditionssalze davon.

2. Verbindung der allgemeinen Formel II

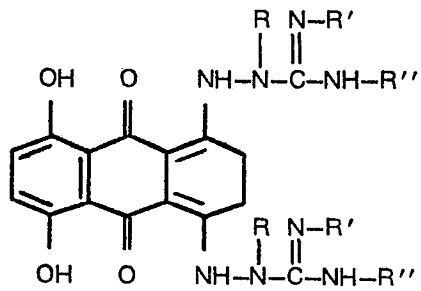

(II)

worin R, R' und R'' ausgewählt sind aus der Gruppe bestehend aus einem Wasserstoffatom und geradkettigen oder verzweigtkettigen $C_1$—$C_4$-Alkylgruppen und R' und R'' zusammen für eine Alkylengruppe $-(CH_2)_n$, wobei n eine ganze Zahl von 2 bis 4 bedeutet, stehen, und die pharmazeutisch verträglichen Säureadditionssalze davon.

3. 1,1'-(5,8-Dihydroxy-1,4-anthrachinonylendiimino)diguanidin-dihydrochlorid.

4. 1,1'-(5,8-Dihydroxy-1,4-anthrachinonylendiimino)-3,3'-dimethyldiguanidin-dihydrochlorid.

5. 1,1'-(5,8-Dihydroxy-1,4-anthrachinonylendiimino)-3,3'-di(1-butyl)-diguanidin-dihydrochlorid.

6. Leuco-5,8-dihydroxy-1,4-bis[2-(2-imidazolin-2-yl]hydrazino]anthrachinon.

7. Leuco-5,8-dihydroxy-1,4-bis[2-(2-imidazolin-2-yl)-2-methylhydrazino]-anthrachinon.

8. Leuco - 5,8 - dihydroxy - 1,4 - bis[2 - (4,5,6,7 - tetrahydro - 1H - 1,3 - diazepin - 2 - yl)hydrazino]anthrachinon.

**Revendications**

1. Un composé choisi dans le groupe consistant en ceux de formule:

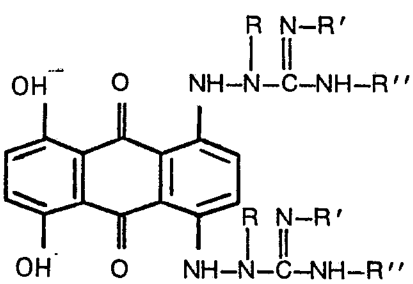

(I)

dans laquelle R, R' et R'' sont choisis dans le groupe consistant en l'hydrogène et les groupes alkyle inférieurs (en $C_1$—$C_4$) à chaîne droite ou ramifiée et R' et R'', pris ensemble, représentent un groupe alkylène —$(CH_2)$—$_n$, dans lequel n est un nombre allant de 2 à 4 inclus, et leurs sels formés par addition avec des acides acceptables pour l'usage pharmaceutique.

2. Un composé choisi dans le groupe consistant en ceux de formule:

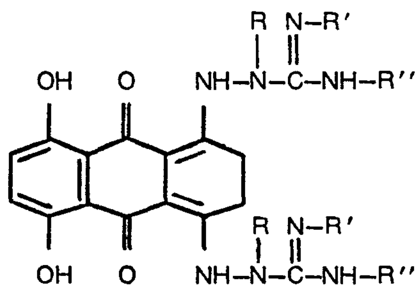

(II)

dans laquelle R, R′ et R″ sont choisis dans le groupe consistant en l'hydrogène et les groupes alkyle inférieurs (en $C_1$—$C_4$) à chaîne droite un ramifiée et R′ et R″, pris ensemble, représentent un groupe alkylène $-(CH_2)_n$, dans lequel n est un nombre allant de 2 à 4 inclus, et leurs sels formés par addition avec des acides acceptables pour l'usage pharmaceutique.

3. Le composé selon la revendication 1, dichlorhydrate de la 1,1′-(5,8-dihydroxy-1,4-anthraquinonylène-diimino)-diguanidine.

4. Le composé selon la revendication 1, dichlorhydrate de la 1,1′-(5,8-dihydroxy-1,4-anthraquinonylène-diimino)-3,3′-diméthyl-diguanidine.

5. Le composé selon la revendication 1, dichlorhydrate de la 1,1′-(5,8-dihydroxy-1,4-anthraquinonylène-diimino)-3,3′-di-(1-butyl)-diguanidine.

6. Le composé selon la revendication 2, leuco-5,8-dihydroxy-1,4-bis[2-(2-imidazoline-2-yl)-hydrazino]-anthraquinone.

7. Le composé selon la revendication 2, leuco-5,8-dihydroxy-1,4-bis[2-(2-imidazoline-1-yl)-2-méthylhydrazino]-anthraquinone.

8. Le composé selon la revendication 2, leuco-5,8-dihydroxy-1,4-bis[2-(4,5,6,7-tétrahydro-1H-1,3-diazépine-2-yl)-hydrazino]-anthraquinone.